# EUROPEAN PATENT APPLICATION

(11) **EP 3 178 431 A1**
(43) Date of publication of application: **14.06.2017**
(21) Application number: 16202673.6
(22) Date of filing: 07.12.2016
(51) Int. Cl.: A61B 18/14, A61B 18/00

(54) **ABLATING AND SENSING ELECTRODES**

(30) Priority: 08.12.2015 US 201514962831
(71) Applicant: Biosense Webster (Israel) Ltd., 2066717 Yokneam (IL)
(72) Inventor: GOVARI, Assaf, 2066717 Yokneam (IL)
(74) Representative: Carpmaels & Ransford LLP

(57) **Abstract**

Apparatus and methods are described, including an expandable structure and one or more sets of electrodes disposed on the surface of the expandable structure. Each one of the sets comprises an ablating electrode, and at least one sensing electrode that is electrically isolated from the ablating electrode. The sensing electrode is contained within the ablating electrode. Other embodiments are also described.

## Description

### FIELD OF THE INVENTION

The present invention relates to ablating and sensing electrodes that may be used, for example, for cardiac ablations.

### BACKGROUND

Cardiac arrhythmias, such as atrial fibrillation, occur when regions of cardiac tissue abnormally conduct electric signals to adjacent tissue, thereby disrupting the normal cardiac cycle and causing asynchronous rhythm.

Procedures for treating arrhythmia include surgically disrupting the origin of the signals causing the arrhythmia, as well as disrupting the conducting pathway for such signals. By selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

Circumferential lesions at or near the ostia of the pulmonary veins have been created to treat atrial arrhythmias. U.S. Patent Nos. 6,012,457 and 6,024,740, both to Lesh, disclose a radially expandable ablation device, which includes a radiofrequency electrode. Using this device, it is proposed to deliver radiofrequency energy to the pulmonary veins in order to establish a circumferential conduction block, thereby electrically isolating the pulmonary veins from the left atrium.

U.S. Patent No. 6,814,733 to Schwartz et al., which is commonly assigned herewith and herein incorporated by reference, describes a catheter introduction apparatus having a radially expandable helical coil as a radiofrequency emitter. In one application the emitter is introduced percutaneously, and transseptally advanced to the ostium of a pulmonary vein. The emitter is radially expanded, which can be accomplished by inflating an anchoring balloon about which the emitter is wrapped, in order to cause the emitter to make circumferential contact with the inner wall of the pulmonary vein. The coil is energized by a radiofrequency generator, and a circumferential ablation lesion is produced in the myocardial sleeve of the pulmonary vein, which effectively blocks electrical propagation between the pulmonary vein and the left atrium.

Another example is found in U.S. Patent No. 7,340,307 to Maguire, et al., which proposes a tissue ablation system and method that treats atrial arrhythmia by ablating a circumferential region of tissue at a location where a pulmonary vein extends from an atrium. The system includes a circumferential ablation member with an ablation element and includes a delivery assembly for delivering the ablation member to the location. The circumferential ablation member is generally adjustable between different configurations to allow both the delivery through a delivery sheath into the atrium and the ablative coupling between the ablation element and the circumferential region of tissue.

### SUMMARY OF THE INVENTION

There is provided, in accordance with some embodiments of the present invention, apparatus that may be used, for example, for ablating cardiac tissue. The apparatus includes an expandable structure. One or more sets of electrodes are disposed on the surface of the expandable structure, each of the sets including an ablating electrode and at least one sensing electrode. The sensing electrode is electrically isolated from the ablating electrode, and is contained within the ablating electrode.

In some embodiments, the sensing electrode is fully contained within the ablating electrode.

In some embodiments, the expandable structure includes a balloon.

In some embodiments, the one or more sets of electrodes consist of 1-5 sets of electrodes.

In some embodiments, a surface area of the ablating electrode is 1-50 mm2.

In some embodiments, a surface area of the sensing electrode is 1-10 mm2.

In some embodiments, a ratio of a surface area of the ablating electrode to a surface area of the sensing electrode is between 1 and 10.

In some embodiments, the ablating electrode is not cylindrical.

In some embodiments, the ablating electrode is planar.

In some embodiments, the sensing electrode is not annular.

In some embodiments, the expandable structure is not planar.

In some embodiments, the expandable structure is ellipsoidal.

In some embodiments, the expandable structure is spherical.

In some embodiments, the sets of electrodes are circumferentially distributed along the surface of the expandable structure.

In some embodiments, the apparatus further includes one or more printed circuit boards (PCBs) disposed on the surface of the expandable structure, and, for each one of the PCBs, a first conducting layer of the PCB is shaped to define one of the sets of electrodes, and a second conducting layer of the PCB is shaped to define a conducting element that is connected to the sensing electrode.

There is further provided, in accordance with some embodiments of the present invention, a method for use with tissue of a subject. An expandable structure is advanced toward the tissue, and is subsequently expanded. Subsequently, using an ablating electrode disposed on a surface of the expandable structure, an ablating current is driven into the tissue. While driving the ablating current into the tissue, using at least one sensing electrode that is electrically isolated from the ablating electrode and that is contained within the ablating electrode, electrical activity of the tissue is sensed.

In some embodiments, the tissue surrounds an ostium of a pulmonary vein of the subject.

There is further provided, in accordance with some embodiments of the present invention, a method for manufacturing ablating apparatus. One or more sets of electrodes are attached to a surface of an expandable structure, each one of the sets including an ablating electrode, and at least one sensing electrode that is electrically isolated from the ablating electrode and is contained within the ablating electrode.

There is further provided, in accordance with some embodiments of the present invention, a printed circuit board (PCB). The PCB includes a first conductive layer shaped to define (i) an ablating electrode, and (ii) at least one sensing electrode that is electrically isolated from the ablating electrode and is contained within the ablating electrode. The PCB further includes a second conductive layer shaped to define a conductive element that is connected to the sensing electrode.

The present invention will be more fully understood from the following detailed description of embodiments thereof, taken together with the drawings, in which:

### BRIEF DESCRIPTION OF THE DRAWINGS

Fig. 1 is a pictorial illustration of a system for evaluating electrical activity and performing ablative procedures on a heart of a living subject, in accordance with some embodiments of the present invention;
Fig. 2 is a schematic illustration of ablation apparatus, in accordance with some embodiments of the present invention;
Fig. 3 is a schematic illustration of a printed circuit board, in accordance with some embodiments of the present invention; and
Fig. 4 is a flow chart for a method for using ablation apparatus, in accordance with some embodiments of the present invention.

### DETAILED DESCRIPTION OF EMBODIMENTS

### OVERVIEW

While performing an ablation of tissue, sensing electrical activity of the tissue may increase the safety and/or efficiency of the ablation procedure, by providing feedback to the operating physician. For example, if a cessation of electrical activity is observed for a particular portion of the tissue, the application of ablating energy to the particular portion of the tissue may be terminated. Conversely, if a sufficient amount of electrical activity continues to be sensed, the application of ablating energy may be continued. Furthermore, in response to the sensing, the physician may choose appropriate ablation parameters. For example, if a sufficient amount of electrical activity is sensed, the physician may infer that the ablation has thus far been unsuccessful, and may therefore, in response, increase the power of the ablating current.

Embodiments of the present invention facilitate the sensing of electrical activity of tissue during the ablation, by providing sensing electrodes that are contained within the ablating electrodes. Due to the containment of the sensing electrodes within the ablating electrodes, the sensing electrodes are positioned to sense electrical activity at a location on the tissue that is being ablated by the ablation electrode, or is at least mostly surrounded by tissue that is being ablated by the ablation electrode. Hence, the sensing electrodes are able to provide more relevant feedback, relative to if the sensing electrodes were merely positioned next to the ablating electrodes.

### SYSTEM DESCRIPTION

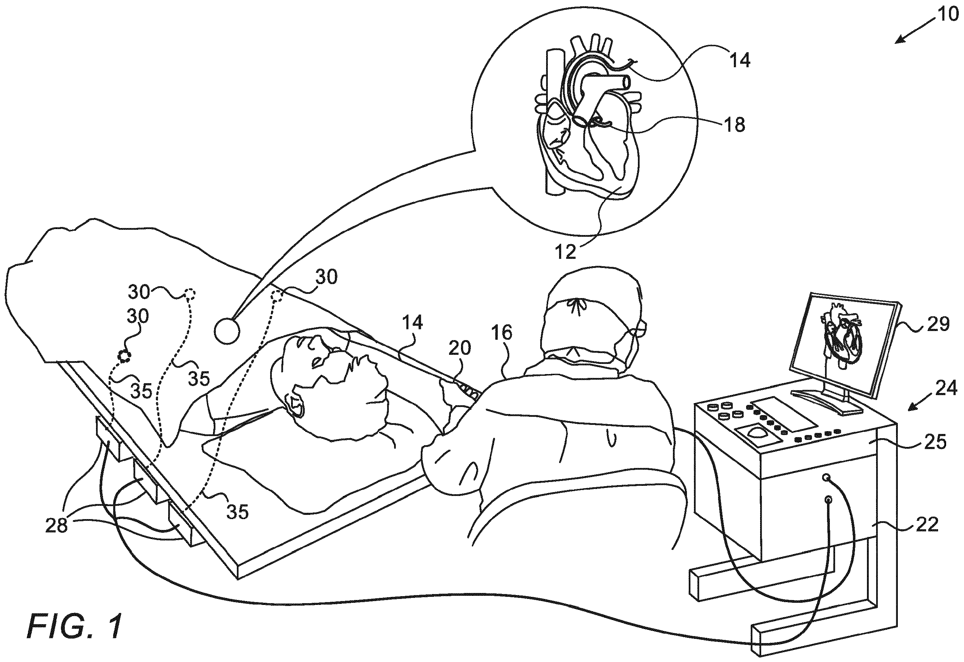

Reference is initially made to Fig. 1, which is a pictorial illustration of a system 10 for evaluating electrical activity and performing ablative procedures on a heart 12 of a living subject, in accordance with some embodiments of the present invention. System 10 comprises a catheter 14, which is percutaneously inserted by an operator 16 through the patient's vascular system into a chamber or vascular structure of heart 12. Operator 16, who is typically a physician, brings the catheter's distal tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091, whose disclosures are herein incorporated by reference. One commercial product embodying elements of system 10 is available as the CARTO^{®} 3 System, available from Biosense Webster, Inc., 3333 Diamond Canyon Road, Diamond Bar, CA 91765. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at distal tip 18, the electrodes applying radiofrequency energy to the myocardium. The energy is absorbed in the tissue, heating it to a point (typically above 60°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

Catheter 14 typically comprises a handle 20, having suitable controls on the handle to enable operator 16 to steer, position and orient the distal end of the catheter as desired for the ablation. To aid operator 16, the distal portion of catheter 14 contains position sensors (not shown) that provide signals to a processor 22, located in a console 24.

Wire connections 35 link console 24 with body surface electrodes 30 and other components of a positioning sub-system for measuring location and orientation coordinates of catheter 14. Processor 22 or another processor (not shown) may be an element of the positioning subsystem. Catheter electrodes (not shown) and body surface electrodes 30 may be used to measure tissue impedance at the ablation site as taught in U.S. Patent No. 7,536,218, issued to Govari et al., which is herein incorporated by reference. Temperature sensors (not shown), e.g., thermocouples and/or thermistors, may be mounted on ablation surfaces on the distal portion of the catheter 14.

Console 24 typically contains one or more ablation power generators 25. Catheter 14 may be adapted to conduct ablative energy to the heart using any known ablation technique, e.g., radiofrequency energy, ultrasound energy, and/or laser-produced light energy. Such methods are disclosed in commonly assigned U.S. Patent Nos. 6,814,733, 6,997,924, and 7,156,816, which are herein incorporated by reference.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent No. 7,756,576, which is hereby incorporated by reference, and in the above-noted U.S. Patent No. 7,536,218.

As noted above, catheter 14 is coupled to console 24, which enables operator 16 to observe and regulate the functions of the catheter 14. Console 24 typically includes a computer with appropriate signal processing circuits, along with processor 22. The processor is coupled to drive a monitor 29. The signal processing circuits typically receive, amplify, filter, and digitize signals from catheter 14, including signals generated by sensors such as electrical, temperature, and contact force sensors, and/or signals generated by a plurality of location sensing electrodes (not shown) located distally in catheter 14. The digitized signals are received and used by the console 24 and the positioning system to compute the position and orientation of catheter 14, and to analyze the electrical signals from the electrodes.

In order to generate electroanatomic maps, processor 22 typically comprises an electroanatomic map generator, an image registration program, an image or data analysis program and a graphical user interface configured to present graphical information on monitor 29.

Typically, system 10 includes other elements, which are not shown in the figures for the sake of simplicity. For example, system 10 may include an electrocardiogram (ECG) monitor, coupled to receive signals from one or more body surface electrodes, in order to provide an ECG synchronization signal to console 24. As mentioned above, system 10 typically also includes a reference position sensor, either on an externally-applied reference patch attached to the exterior of the subject's body, or on an internally-placed catheter, which is inserted into heart 12 maintained in a fixed position relative to heart 12. Conventional pumps and lines for circulating liquids through catheter 14 for cooling the ablation site are provided. System 10 may receive image data from an external imaging modality, such as an MRI unit or the like, and includes image processors that can be incorporated in or invoked by the processor 22 for generating and displaying images.

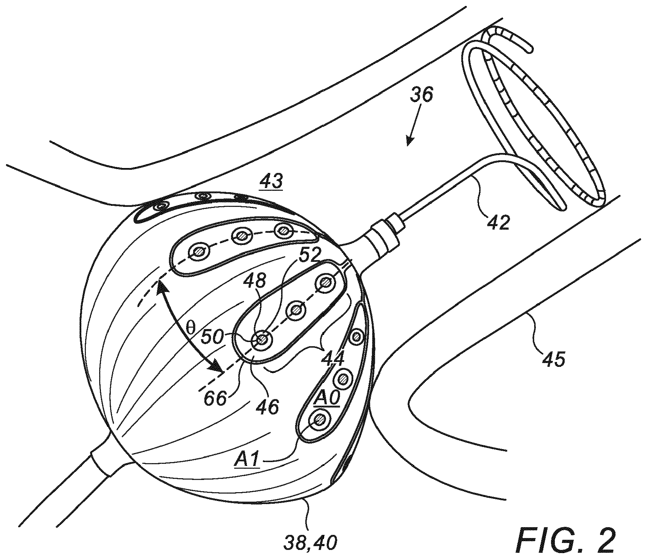

Reference is now made to Fig. 2, which is a schematic illustration of ablation apparatus 36, in accordance with some embodiments of the present invention. Apparatus 36 comprises an expandable structure 38, such as an inflatable balloon 40 or an expandable basket. One or more sets 44 of electrodes (e.g., 1-5 sets of electrodes) are disposed on the surface of expandable structure 38, e.g., by being formed on a printed circuit board (PCB) 66 that is disposed on the surface of expandable structure 38. Each one of sets 44 comprises an ablating electrode 46, and one or more (e.g., at least one and/or fewer than five, e.g., three) sensing electrodes 48.

Typically, sets 44 are circumferentially distributed (e.g., evenly distributed) along the surface of the expandable structure. For example, as shown in the figure, the sets may be circumferentially distributed such that the angle theta between each pair of successive sets is between 30 and 180 degrees. The circumferential distribution of the sets of electrodes facilitates ablation of tissue that surrounds the pulmonary ostium 43, in that, by activating each of the ablating electrodes in sequence, the full circumference of the tissue may be ablated without substantially moving the apparatus.

In general, when sensing electrical activity of tissue to provide feedback for an ablation procedure, as described hereinabove, it is advantageous to position the sensing electrodes as close as possible to the portion of tissue that is being ablated. For example, while an ablating electrode is ablating at "12 o'clock" with respect to the pulmonary ostium, it is better to sense the electrical activity of tissue located at "12 o'clock," rather than tissue located at "12:30" or "11:30." Embodiments of the present invention provide such an advantage, in that, as shown in the figure, each sensing electrode 48 is contained within an ablating electrode 46.

In the context of the claims and description of the present application, the "containment" of each sensing electrode within an ablating electrode means that (i) most (e.g., more than 75%, such as more than 90%) of a perimeter 50 and/or an area A1 of the sensing electrode is contained within the ablating electrode, and/or (ii) the sensing electrode is positioned to sense electrical activity at a location on the tissue that is being ablated by the ablation electrode, or is at least mostly surrounded by tissue that is being ablated by the ablation electrode. For example, as shown in the figure, each sensing electrode may be fully contained within an ablating electrode.

As shown in Fig. 2, the sensing electrodes are typically distributed both (i) circumferentially (by virtue of being contained within the circumferentially-distributed ablating electrodes), and (ii) "lengthwise" along each ablating electrode. Such a configuration increases the likelihood that a plurality of sensing electrodes will the contact the ostium at a plurality of different points, regardless of the orientation or exact positioning of the expandable structure. Hence, such a configuration facilitates a more accurate and/or precise assessment of the electrical activity of the tissue.

To prevent crosstalk between the ablating electrode and the sensing electrode that is contained therein, the sensing electrode is electrically isolated from ablating electrode 46. For example, Fig. 2 shows an annular "moat" 52, comprising electrically non-conductive material, that isolates the sensing electrode from the ablating electrode. (The sensing electrode is thus disposed as an "island" within the ablating electrode.)

The expandable structure is typically not planar. For example, as shown in the figure, the expandable structure may be ellipsoidal (e.g., spherical, oblate spheroidal, or prolate spheroidal). The ellipsoidal shape of the expandable structure facilitates bringing the expandable structure into contact with pulmonary ostium 43, as well as ablating the tissue surrounding the ostium.

Typically, the surface area A0 of each ablating electrode (which does not include the total surface area of any contained sensing electrodes) is 1-50 mm2, and/or surface area A1 of each sensing electrode is 1-10 mm2. Alternatively or additionally, the ratio of A0 to A1 may be between 1 and 10. The ablating electrodes and sensing electrodes are typically not cylindrical or annular.

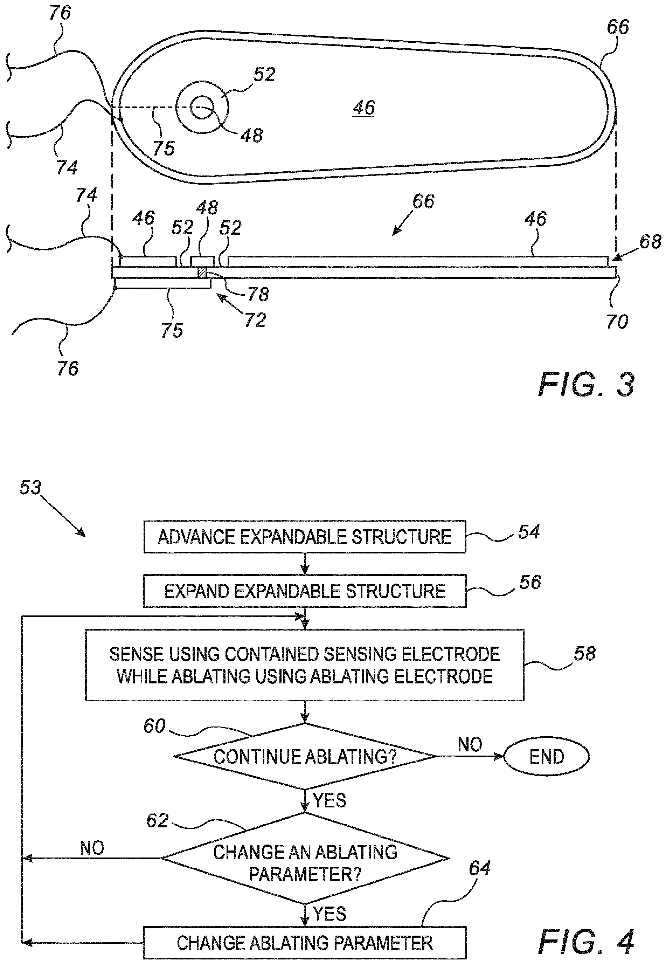

Reference is now additionally made to Fig. 3, which is a schematic illustration of printed circuit board (PCB) 66, in accordance with some embodiments of the present invention. The top and bottom of the figure show top and side views of PCB 66, respectively. PCB 66 typically has at least two conducting layers. A first conducting layer 68 of PCB 66 is shaped to define set 44 of electrodes, comprising ablating electrode 46 and sensing electrode(s) 48. (For simplicity, Fig. 3 shows an embodiment having only one sensing electrode.) A wire 74 connects the ablating electrode to apparatus outside of the subject's body, e.g., to console 24, via catheter 14.

A second conducting layer 72 of the PCB is shaped to define a conducting element 75 (which may be referred to as a trace) that is connected to the sensing electrode. A wire 76 connects conducting element 75 to apparatus outside of the subject's body, e.g., to console 24, via catheter 14. Typically, a dielectric layer 70 is disposed between first conducting layer 68 and second conducting layer 72, and conducting element 75 connects to the sensing electrode via a plated hole 78 (sometimes referred to as a "via") or other electrically-conductive interconnection through dielectric layer 70.

In some embodiments, PCB 66 does not comprise second conducting layer 72 or conducting element 75, and wire 76 connects directly to the sensing electrode via plated hole 78. Moat 52 is formed by etching an annulus around the sensing electrode, such that the surface of the dielectric layer constitutes a non-conductive barrier between the sensing electrode and the ablating electrode.

Typically, PCB 66 is at least somewhat flexible. The flexibility of PCB 66 helps the PCB fit inside the catheter before and after the procedure, and/or helps the PCB conform to the surface of expandable structure 38 when the expandable structure is expanded. In some embodiments, PCB 66 flexes relatively little when the expandable structure is expanded, such that PCB 66 is planar even when disposed on the surface of the expanded expandable structure. Likewise, the ablating electrodes and/or sensing electrodes may be planar, even when disposed on the surface of the expanded expandable structure.

Reference is now additionally made to Fig. 4, which is a flow chart for a method 53 for using apparatus 36, in accordance with some embodiments of the present invention. At an advancing step 54, expandable structure 38 is advanced toward the tissue that is to be ablated. (For example, expandable structure 38 may be deployed from the distal end of catheter 14 (Fig. 1), following the deployment of the catheter into the left atrium of the heart.) Subsequently, at an expanding step 56, the expandable structure is expanded, and one or more (e.g., all) of the ablating electrodes are brought into contact with (e.g., pressed against) the tissue.

Subsequently, at an ablating-and-sensing step 58, at least one sensing electrode that is contained within an ablating electrode is used to sense electrical activity of the tissue, while the ablating electrode is used to drive an ablating current into the tissue. In response to the sensed electrical activity, at a first decision step 60, the operating physician decides whether to continue ablating. If the physician decides to continue ablating (e.g., in response to sensing that the tissue remains electrically active), method 53 proceeds to a second decision step 62, at which the physician decides whether to change any of the ablating parameters (e.g., an amplitude of the ablating current). If the physician decides to change a parameter, the parameter is changed, at a parameter-changing step 64. Ablating-and-sensing step 58 is then repeated, until the physician is satisfied that the tissue has been sufficiently ablated.

For ablation of tissue surrounding an ostium 43 of a pulmonary vein 45, method 53 is typically repeatedly performed, whereby, for each performance of method 53, a different set of electrodes is used, such that the tissue is ablated "around the clock."

Typically, the signal processing circuits described above with reference to Fig. 1 are used to filter the signals received from the sensing electrodes, such that (i) the lower-frequency portion of the signals, corresponding to electrical activity of the tissue, is retained, while (ii) the higher-frequency portion of the signals, corresponding to the radiofrequency ablation signal, is discarded.

The scope of the present invention further includes a method for manufacturing apparatus 36, by attaching one or more sets 44 of electrodes to a surface of expandable structure 38.

Apparatus and techniques described herein may be practiced in combination with apparatus and techniques described in U.S. Patent Application 14/578,807 to Govari, which is incorporated herein by reference. For example, Fig. 2 shows a guide 42, which may be used to stabilize apparatus 36 by engaging the interior wall of pulmonary vein 45, as described in '807 to Govari. Alternatively or additionally, as described in '807 to Govari, expandable structure 38 may be fenestrated, such that an irrigating fluid (e.g., saline) may be passed out of the expandable structure during the ablation.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

### Aspect of the invention

1. A method for use with tissue of a subject, the method comprising:
   advancing an expandable structure toward the tissue;
   subsequently, expanding the expandable structure;
   subsequently, using an ablating electrode disposed on a surface of the expandable structure, driving an ablating current into the tissue; and
   while driving the ablating current into the tissue, using at least one sensing electrode that is electrically isolated from the ablating electrode and that is contained within the ablating electrode, sensing electrical activity of the tissue.
2. The method according to aspect 1, wherein the tissue surrounds an ostium of a pulmonary vein of the subject.

## Claims

1. Apparatus comprising:
an expandable structure; and
one or more sets of electrodes disposed on a surface of the expandable structure, each one of the sets comprising:
an ablating electrode; and
at least one sensing electrode that is electrically isolated from the ablating electrode, the sensing electrode being contained within the ablating electrode.

2. The apparatus according to claim 1, wherein the sensing electrode is fully contained within the ablating electrode.

3. The apparatus according to claim 1, wherein the expandable structure comprises a balloon.

4. The apparatus according to claim 1, wherein the one or more sets of electrodes consist of 1-5 sets of electrodes.

5. The apparatus according to claim 1, wherein a surface area of the ablating electrode is 1-50 mm2, preferably 1-10 mm2.

6. The apparatus according to claim 1, wherein a ratio of a surface area of the ablating electrode to a surface area of the sensing electrode is between 1 and 10.

7. The apparatus according to claim 1, wherein the ablating electrode is not cylindrical, or is planar.

8. The apparatus according to claim 1, wherein the sensing electrode is not annular.

9. The apparatus according to claim 1, wherein the expandable structure is not planar.

10. The apparatus according to claim 1, wherein the expandable structure is ellipsoidal.

11. The apparatus according to claim 10, wherein the expandable structure is spherical.

12. The apparatus according to claim 1, wherein the sets of electrodes are circumferentially distributed along the surface of the expandable structure.

13. The apparatus according to claim 1, further comprising one or more printed circuit boards (PCBs) disposed on the surface of the expandable structure, wherein, for each one of the PCBs, a first conducting layer of the PCB is shaped to define one of the sets of electrodes, and a second conducting layer of the PCB is shaped to define a conducting element that is connected to the sensing electrode.

14. A method comprising:
attaching one or more sets of electrodes to a surface of an expandable structure, each one of the sets including:
an ablating electrode, and
at least one sensing electrode that is electrically isolated from the ablating electrode and is contained within the ablating electrode.

15. A printed circuit board (PCB), comprising:
a first conductive layer shaped to define:
an ablating electrode, and
at least one sensing electrode that is electrically isolated from the ablating electrode and is contained within the ablating electrode; and
a second conductive layer shaped to define a conductive element that is connected to the sensing electrode.
